# EUROPEAN PATENT APPLICATION

(11) **EP 4 063 514 A1**
(43) Date of publication of application: **28.09.2022**
(21) Application number: 20889543.3
(22) Date of filing: 04.11.2020
(51) Int. Cl.: C12Q 1/42, G01N 33/53, G01N 33/543

(54) **MEASUREMENT METHOD USING ANTI-IMMUNOCOMPLEX ANTIBODY**

(30) Priority: 20.11.2019 JP 2019209407; 29.11.2019 JP 2019216521; 12.03.2020 JP 2020042915; 10.06.2020 JP 2020100842; 18.08.2020 JP 2020138101
(71) Applicant: Tosoh Corporation, Syunan-shi, Yamaguchi 746-8501 (JP)
(72) Inventor: MUTO, Yu, Ayase-shi, Kanagawa 252-1123 (JP); MISAWA, Kouichi, Ayase-shi, Kanagawa 252-1123 (JP)
(74) Representative: Novagraaf International SA
(86) International application number: PCT/JP2020/041145
(87) International publication number: WO 2021/100459

(57) **Abstract**

The invention provides an assay method for an assay system using an anti-immunocomplex antibody, which is able to increase reaction efficiency. The n immunoassay method uses:
• an anti-hapten rabbit monoclonal antibody immobilized on a water-insoluble carrier, and
• a labeled anti-immunocomplex antibody,
wherein the method comprises the following steps (i) to (iii):
(i) a step of reacting the anti-hapten rabbit monoclonal antibody immobilized on the water-insoluble carrier with a hapten in a solution to be measured,
(ii) a step of reacting the labeled anti-immunocomplex antibody with the hapten-anti-hapten rabbit monoclonal antibody immune complex, and
(iii) a step of detecting the signal from the label.

## Description

### FIELD

The present invention relates to a measurement method using an anti-immunocomplex antibody.

### BACKGROUND

Haptens are low molecular compounds that are commonly measured by the method of immunoassay known as competitive assay. A competitive assay is a method in which a hapten included in a sample and a labeled hapten that is labeled with a radioactive isotope or enzyme are reacted in a competitive manner against a fixed amount of anti-hapten antibody. A large amount of hapten in a sample lowers the amount of labeled hapten that binds with the anti-hapten antibody. This allows the amount of hapten in the sample to be estimated based on the proportion of labeled hapten binding with the anti-hapten antibody. The measurement sensitivity in a competitive assay depends on the affinity constant of the anti-hapten antibody used. However it is difficult to obtain anti-hapten antibodies with high affinity constants, making it very difficult to measure trace amounts of haptens (NPL 1).

One method for solving this problem of competitive immunoassay has been proposed, as a non-competitive immunoassay method using an anti-immunocomplex antibody. Hapten assay systems using anti-immunocomplex antibodies can measure haptens in samples in a non-competitive manner. Examples of this method are as follows.
(1) An anti-immunocomplex antibody is immobilized on a plate or the like.
(2) After mixing a sample containing a target hapten with enzyme-labeled anti-hapten antibody, the mixture is added to (1).
(3) The excess labeled anti-hapten antibody is washed off and separated.
(4) Substrate for the labeling enzyme is added and the signal from the enzyme-substrate reaction is detected.

Since the anti-immunocomplex antibody selectively binds to immune complexes of the hapten and anti-hapten antibody, increase in the signal is observed as the amount of hapten in the sample increases.

A specific example of measurement using an anti-immunocomplex antibody is the estradiol (E2) assay system described in PTL 1. In the technique disclosed in PTL 1, enzyme-labeled anti-E2 antibody and a sample to be measured E2 are mixed beforehand by a common method and the mixture is subsequently reacted with a carrier having the anti-immunocomplex antibody immobilized.

In the method described in PTL 1, anti-immunocomplex mouse monoclonal antibody is immobilized on a solid phase carrier. Since the reaction between the immobilized antibody on a solid phase and antigen takes place in the heterogeneous system of a solid-liquid interface, the reaction efficiency is low (NPL 2). When mouse monoclonal antibody with low affinity is used in a solid phase, it is thought that the reaction at the solid-liquid interface becomes a bottleneck lowering the overall reaction efficiency.

Also, in an assay system in which previously labeled anti-hapten antibody and an assay sample are mixed and the immune complex is reacted with an anti-immunocomplex antibody immobilized on a solid phase carrier as described in NPL 1, the washing step is a single step process. As mentioned in NPL 3, false highs or false lows have been reported in single step assays due to substances copresent in assay solutions.

### [CITATION LIST]

### [NON PATENT LITERATURE]

[NPL 1] Bunseki, 551-552; 2004
[NPL 2] Soh, N., Ishida, Y., Kume M., Imato, T., Summary of the 53rd Meeting of The Japan Society for Analytical Chemistry (2004), Development of rapid and convenient antigen-antibody reaction assay methods [P3093]
[NPL 3] Adachi, Y., Ogawa, H., Tosaoka, N., Mukoyama, K., Summary of the 67th Meeting of the Japan Association of Medical Technologists (May, 2018)
[NPL 4] Takagi, R., Analysis of molecular interaction of proteins, Protein activity and molecular function, Kagaku Dojin, 2009, p.29-51

### [PATENT LITERATURE]

[PTL 1] Japanese Patent No. 6221466

### SUMMARY

### [TECHNICAL PROBLEM]

It is an object of the present invention to provide an assay method for an assay system using an anti-immunocomplex antibody, which is able to increase reaction efficiency.

### [SOLUTION TO PROBLEM]

In light of the problems of the prior art described above, the present invention provides the following aspects.
(1) An immunoassay method using:
   - an anti-hapten rabbit monoclonal antibody immobilized on a water-insoluble carrier, and
   - a labeled anti-immunocomplex antibody,
   wherein the method comprises the following steps (i) to (iii):
   (i) a step of reacting the anti-hapten rabbit monoclonal antibody immobilized on the water-insoluble carrier with a hapten in a solution to be measured,
   (ii) a step of reacting the labeled anti-immunocomplex antibody with the hapten-anti-hapten rabbit monoclonal antibody immune complex, and
   (iii) a step of detecting the signal from the label.
(2) The method according to (1), wherein steps (i), (ii) and (iii) are carried out in that order, and the method has washing steps between steps (i) and (ii) and between steps (ii) and (iii).
(3) The method according to (1) or (2), wherein the anti-immunocomplex antibody is a mouse monoclonal antibody.
(4) The method according to any one of (1) to (3), wherein the label is alkaline phosphatase.
(5) The method according to any one of (1) to (4), wherein the hapten is estradiol, thyroxine or digoxin.
(6) The method according to any one of (1) to (5), wherein step (i) is carried out in the presence of an absorption antibody.

The present invention will now be explained in greater detail.

### (1) Hapten

Usually the hapten of the invention is not particularly restricted so long as it is a substance of low molecular weight that can be measured by competitive assay, and examples include thyroid hormones such as triiodothyronine, thyroxine or 3,5-diiodo-L-thyronine, steroid hormones such as estrone, estradiol (E2), estriol, progesterone or cortisol, and steroid glycosides such as digoxin (Dig). Steroid hormones and steroid glycosides are particularly preferred for the invention, among which E2 and Dig are especially preferred. Thyroxine is preferred among thyroid hormones.

### (2) Solution to be measured

The solution to be measured is not particularly restricted so long as it comprises a hapten to be assayed. For example, human blood (whole blood, blood plasma or serum) or body fluids such as urine are suitable.

### (3) Anti-immunocomplex antibody

The anti-immunocomplex antibody is not an antibody for a hapten nor an antibody for an antibody for a hapten, but rather it is an antibody for a complex between a hapten and an antibody for it. According to the invention, the anti-immunocomplex antibody is preferably obtained from an animal species other than a rabbit, from the viewpoint of obtaining antibodies specific for the complex of the anti-hapten rabbit monoclonal antibody and hapten. Examples of suitable animal species include mouse, rat, goat and sheep. Anti-immunocomplex monoclonal antibodies obtained from mice are especially preferred for the invention.

### (4) Anti-hapten rabbit monoclonal antibody

According to the invention, rabbit monoclonal antibody is used as the anti-hapten antibody. This is because rabbit monoclonal antibodies have higher affinity for haptens than mouse monoclonal antibodies. The affinity is preferably a dissociation constant (KD) of 10⁻¹⁰ (M) or lower and more preferably 10⁻¹¹ (M) or lower.

Since the anti-hapten antibody is to be used as a solid phase antibody for the invention, the antibody used must be rabbit monoclonal antibody with high affinity. As described in NPL 4, antigen that has once bound with a solid phase surface antibody is able to re-bind with nearby solid phase antibody after having been temporarily dissociated by a washing procedure or the like, so long as it is a protein (polymer) with a slow diffusion rate. Because the antigen of the invention is a low-molecular hapten, however, because of its high diffusion rate it cannot easily rebind with antibody on a solid phase surface after having been dissociated from antibody by washing procedures. According to the invention it is essential to use a high-affinity rabbit monoclonal antibody as the solid phase antibody in order to inhibit dissociation of hapten from the solid phase antibody.

### (5) Water-insoluble carrier

The water-insoluble carrier of the invention is not particularly restricted and may be microparticles, particles, beads or a plate of a resin, glass or polystyrene. The anti-hapten rabbit monoclonal antibody is immobilized on the water-insoluble carrier. The method of immobilization is not particularly restricted and may be direct immobilization or indirect immobilization (by avidin-biotin bonding, for example).

### (6) Labeling

The label used for the invention is not particularly restricted, and an enzyme, radioactive isotope or pigment, for example, may be used. Alkaline phosphatase is preferred for use as the enzyme. The label is bonded to the anti-immunocomplex antibody. The method of labeling is not particularly restricted and may be direct labeling or indirect labeling (by avidin-biotin bonding, for example).

### (7) Steps (i) to (iii)

Steps (i) to (iii) are preferably carried out in that order, although steps (i) and (ii) may be carried out simultaneously. Another step may also be inserted between steps (i) and (ii) or between steps (ii) and (iii). For example, the following washing step may be used to increase the measuring sensitivity.

### (8) Washing step

The washing step is a step of washing the components that have been nonspecifically adsorbed onto the water-insoluble carrier. The antigen-antibody reaction can be inhibited by co-present substances if the solution to be measured contains significant amounts of contaminants such as blood. It has also been reported that assay systems often do not function properly when other substances are present. Carrying out a washing step can reduce the effects of co-present substances even in such cases. In order to reduce the effects of co-present substances it is particularly effective to carry out washing steps between steps (i) and (ii) and between steps (ii) and (iii), or in other words, to conduct the assay by a 2-step method with a total of two washing steps (NPL 3).

### (9) Assay method

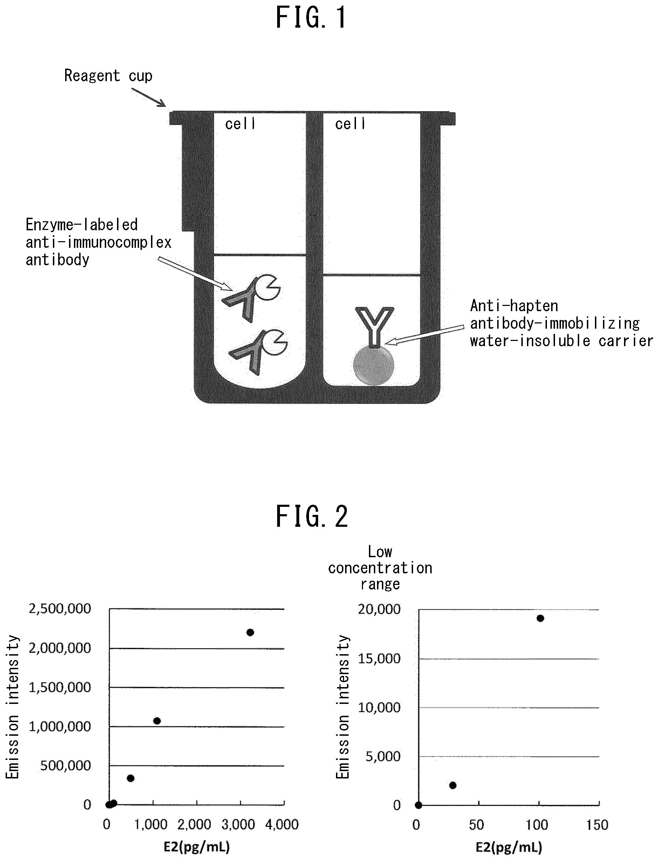

In the method of the invention it is preferred to use a fully automatic immunoassay device such as described in Seibutsu Shiryo Bunseki, Vol. 39, No. 4 (2016). For example, it is preferred to conduct the following automatic assay method using a reagent cup having two cells as shown in Fig. 1.
(9-1) A water-insoluble carrier immobilizing the anti-hapten antibody is dispensed into one cell of the two-cell cup while enzyme-labeled anti-immunocomplex antibody is dispensed into the other cell, for use as a reagent cup.
(9-2) The reagent cup and solution to be measured are set in an automatic immunoassay device.
(9-3) In the automatic immunoassay device, the solution to be measured containing the hapten is dispensed into the cell on the water-insoluble carrier side of the reagent cup and reacted.
(9-4) Upon completion of the reaction, the components that have not been adsorbed onto the anti-hapten antibody-immobilizing water-insoluble carrier are washed off.
(9-5) The enzyme-labeled anti-immunocomplex antibody in the reagent cup is transferred to the cell on the water-insoluble carrier side.
(9-6) Upon completion of the reaction, the components that have not been adsorbed onto the anti-hapten antibody-immobilizing water-insoluble carrier are washed off.
(9-7) Enzyme substance is added, and the emission intensity of the product of the enzyme reaction is measured.

### (10) Cross-reaction

A cross-reaction is a reaction between an antibody for a substamce to be measured and a substance similar to the substance to be measured (cross-reactive substance). In immunoassay reagents, when a measurement system with high cross-reactivity is used, the measurement result will be falsely high due to the cross-reaction. The inventors found that when haptens, which are usually measured using a competitive method, are subjected to a sandwich assay using anti-immunocomplex antibodies, the cross reactivity may be greater in the sandwich assay. The inventors investigated this point and found that one of the reasons for this is that the sandwich assay uses more anti-hapten antibodies than the competitive method. Another possible reason was that the surface structures of the immunocomplexes of the anti-hapten antibody and hapten and the complex of the anti-hapten antibody and the cross-reactive substance were identical, and the anti-immunocomplex antibody would recognize both.

### (11) Absorption antibody

An absorption antibody is an antibody that reacts specifically with cross-reactive substances but essentially does not react with the hapten to be measured. While the antibody preferably has absolutely no reactivity for the hapten to be measured, there is no problem with weak reactivity that does not adversely affect the measurement sensitivity. Specifically, the reactivity of the absorption antibody for the hapten to be measured is preferably about ≤1/100 of the reactivity for the cross-reactive substance.

### [ADVANTAGEOUS EFFECTS OF INVENTION]

Highly sensitive assay systems are desirable for haptens measured using anti-immunocomplex antibodies. In the assay method of the invention, the water-insoluble carrier used has high-affinity anti-hapten rabbit monoclonal antibody immobilized on a solid phase carrier. It is thereby possible to lower reduction in reaction efficiency at the solid-liquid interface where efficiency is low. By using anti-hapten antibody as a solid phase antibody it is possible to provide an assay system wherein washing steps can be carried out two or more times as necessary after reaction with the hapten in the solution to be measured, thus reducing the effects of co-present substances. It is also possible to provide an assay system with reduced cross-reaction during reaction between the anti-hapten antibody and the hapten to be measured, when absorption antibodies are also present.

### BRIEF DESCRIPTION OF DRAWINGS

Fig. 1 is a drawing showing an example of a reagent cup to be used for the invention.
Fig. 2 is a pair of graphs showing the results for the calibration curve of Example 1(3-2).
Fig. 3 is a pair of graphs showing the results for the calibration curve of Example 2(3-2).
Fig. 4 is a graph showing the S/N ratio for Example 3 and Comparative Example 1.
Fig. 5 is a graph showing the results for the calibration curve of Example 4(3-2).
Fig. 6 is a graph showing the results for the calibration curve of Example 5(4-2).
Fig. 7 is a graph showing the results for the calibration curve of Example 6(2).
Fig. 8 is a graph showing the results for the calibration curve of Example 7(4-2).
Fig. 9 is a graph showing the results for the calibration curve of Example 8(2).

### EXAMPLES

### [Example 1]

An Example of the present invention will now be described in detail, for a method using rabbit monoclonal antibody for estradiol (E2) and anti-immunocomplex mouse monoclonal antibody for the immune complex, with the understanding that the Example is not intended to be restrictive on the invention.

### (1) Anti-E2 rabbit monoclonal antibody

The anti-E2 rabbit monoclonal antibody was obtained by the method described in Japanese Unexamined Patent Publication No. 2009-240300.

### (2) Anti-immunocomplex mouse monoclonal antibody

Antibody for E2/anti-E2 rabbit monoclonal antibody immune complex (anti-immunocomplex mouse monoclonal antibody) was obtained by the method described in Japanese Patent Publication No. 6031944.

### (3) Measurement

Automatic analysis was carried out by the following method using a fully automatic chemiluminescent enzyme immunoassay device (AIA-CL2400 by Tosoh Corp.).

### (3-1) Preparation of reagent cup

A cup having two cells as shown in Fig. 1 was used to prepare a reagent cup for use in automatic analysis. (Hereunder, one cell of the cup will be referred to as the "microparticle cell" and the other as the "conjugate cell", as per the contents.)

A solution containing microparticles immobilizing anti-E2 rabbit monoclonal antibody was dispensed into the microparticle cell. A solution containing β-estradiol-6-one-6-(O-carboxymethyloxime) was dispensed into the conjugate cell together with alkaline phosphatase-labeled anti-immunocomplex mouse monoclonal antibody, with reference to PTL 1. The solution was lyophilized and sealed with aluminum to prepare an E2-assay reagent cup.

### (3-2) Measurement

The E2-assay reagent cup prepared in (3-1) was set in the fully automatic chemiluminescent enzyme immunoassay device (AIA-CL2400 by Tosoh Corp.) and measurement was conducted for 6 different samples (call to cal6) of known E2 concentration to construct a calibration curve. For the measurement, 50 µL of a sample of known concentration and 50 µL of a diluent were dispensed into the microparticle cell of the E2-assay reagent cup and reacted for 5 minutes (first-order reaction), after which washing was performed with a cleaning fluid (B/F separation) and the reagent in the conjugate cell that dissolved in the diluent (50 µL) was transferred to the microparticle cell and reacted (secondary reaction, 3 minutes). After completion of the secondary reaction, a second washing was performed with cleaning fluid. Enzyme substrate was then added and the emission intensity was measured. The measurement results are shown in Table 1 and Fig. 2.

The emission intensity was confirmed to increase in proportion to E2 concentration, making it possible to construct an E2 assay system using anti-immunocomplex antibody.

**[Table 1]**

| | | | | | | |
|---|---|---|---|---|---|---|
| E2 concentration (pg/mL) | 0 | 28.4 | 101 | 490 | 1,090 | 3,220 |
| Emission intensity (cps) | 27 | 2,049 | 19,110 | 339,723 | 1,073,8761 | 2,200,960 |

These results confirmed that E2 can be detected with high sensitivity in an assay system using anti-hapten rabbit monoclonal antibody as a solid phase antibody and anti-immunocomplex antibody as a labeled antibody.

### [Example 2]

An Example of the present invention will now be described in detail, for a method using rabbit monoclonal antibody for thyroxine (FT4) and anti-immunocomplex mouse monoclonal antibody for the immune complex, with the understanding that the Example is not intended to be restrictive on the invention.

### (1) Anti-FT4 rabbit monoclonal antibody

The anti-FT4 rabbit monoclonal antibody was obtained by the method described in Japanese Unexamined Patent Publication No. 2009-240300.

### (2) Anti-immunocomplex mouse monoclonal antibody

The antibody for FT4/anti-FT4 rabbit monoclonal antibody immune complex (anti-immunocomplex mouse monoclonal antibody) was obtained by the method described in Japanese Patent Publication No. 6031944.

### (3) Measurement

Automatic analysis was carried out by the following method using a fully automatic chemiluminescent enzyme immunoassay device (AIA-CL2400 by Tosoh Corp.).

### (3-1) Preparation of reagent cup

A cup having two cells as shown in Fig. 1 was used to prepare a reagent cup for use in automatic analysis. A solution containing microparticles immobilizing anti-FT4 rabbit monoclonal antibody was dispensed in an amount of 50 µL into the microparticle cell. A solution containing alkaline phosphatase-labeled anti-immunocomplex mouse monoclonal antibody was dispensed in an amount of 75 µL into the conjugate cell. This was sealed with aluminum to obtain a reagent cup for FT4 assay.

### (3-2) Measurement

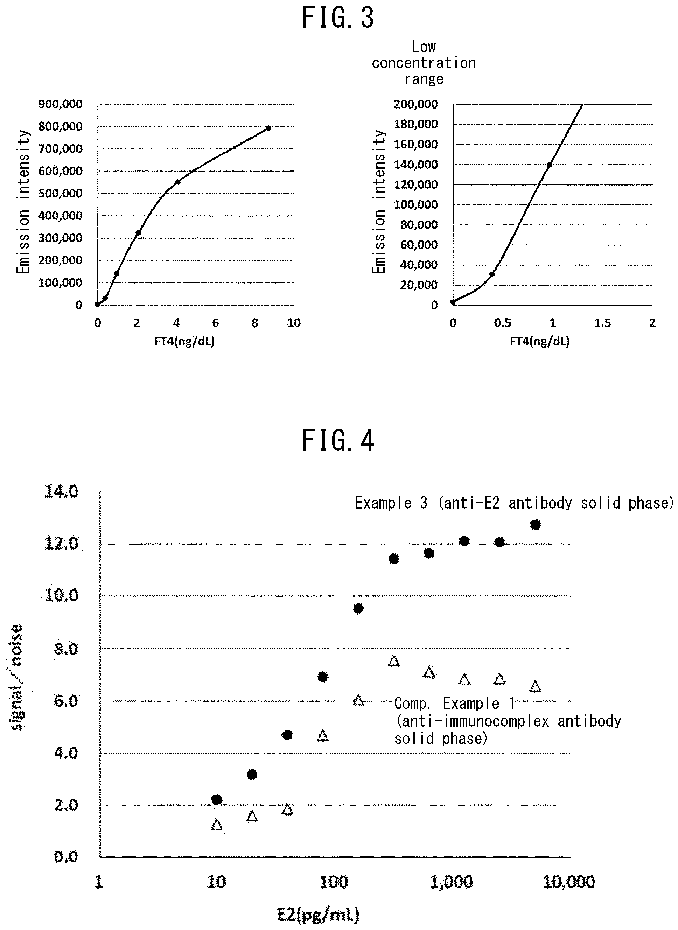

The FT4-assay reagent cup prepared in (3-1) was set in the fully automatic chemiluminescent enzyme immunoassay device (AIA-CL2400 by Tosoh Corp.) and measurement was conducted for 6 different samples (call to cal6) of known FT4 concentration to construct a calibration curve. For the measurement, 5 µL of a sample of known concentration and 5 µL of a diluent were dispensed into the microparticle cell of the FT4-assay reagent cup and reacted for 5 minutes (first-order reaction), after which washing was performed with a cleaning fluid (B/F separation) and the reagent in the conjugate cell (50 µL) was transferred to the microparticle cell and reacted (secondary reaction, 3 minutes). After completion of the secondary reaction, a second washing was performed with cleaning fluid. Enzyme substrate was then added and the emission intensity was measured. The measurement results are shown in Table 2 and Fig. 3.

The emission intensity was confirmed to increase in proportion to FT4 concentration, making it possible to construct an FT4 assay system using anti-immunocomplex antibody.

**[Table 2]**

| | | | | | | |
|---|---|---|---|---|---|---|
| FT4 concentration (ng/dL) | 0 | 0.394 | 0.967 | 2.07 | 4.06 | 8.7 |
| Emission intensity (cps) | 3,105 | 30,926 | 139,586 | 323,633 | 551,883 | 793,500 |

These results confirmed that FT4 can be detected with high sensitivity in an assay system using anti-hapten rabbit monoclonal antibody as a solid phase antibody and anti-immunocomplex antibody as a labeled antibody.

### [Example 3]

An Example of the present invention will now be described in detail, for a method using rabbit monoclonal antibody for estradiol (E2) and anti-immunocomplex mouse monoclonal antibody for the immune complex on a 96-well ELISA plate, with the understanding that the Example is not intended to be restrictive on the invention.

### (1) Anti-E2 rabbit monoclonal antibody

The anti-E2 rabbit monoclonal antibody used was the same antibody as in Example 1.

### (2) Anti-immunocomplex mouse monoclonal antibody

The anti-immunocomplex mouse monoclonal antibody used was the same antibody as in Example 1.

### (3) Measurement

A plate reader (TECAN, infinite F500) was used for measurement of E2 by the following method.

Anti-E2 rabbit monoclonal antibody was prepared as solid phase on a 96-well microplate (black, Greiner) in an amount of 0.25 µg/mL (carbonate buffer (0.05 M, pH 9.6)). Blocking treatment was then carried out with 1% skim milk/PBS. Using a separate plate, an E2 dilution series (2x dilution from 5,000 pg/mL) was prepared and dispensed into an anti-E2 rabbit monoclonal antibody-immobilized 96-well microplate at 100 µL/well. After incubating for 5 minutes, it was washed using a plate washer. Anti-immunocomplex mouse monoclonal antibody labeled with alkaline phosphatase was prepared to an absorbance of 1 mA at 280 nm, and then dispensed at 100 µL/well and incubated for 5 minutes. It was then washed using a plate washer, 4-MUP (4-methylumbelliferyl phosphate, Merck) was added as enzyme substrate and the fluorescence intensity was measured after 30 minutes.

### [Comparative Example 1] Example using anti-immunocomplex antibody as solid phase antibody

A Comparative Example of the invention will now be described in detail, for a method using rabbit monoclonal antibody for estradiol (E2) and anti-immunocomplex mouse monoclonal antibody for the immune complex, with the anti-immunocomplex mouse monoclonal antibody immobilized on a water-insoluble carrier (hereunder referred to as "solid phase antibody"), on a 96-well ELISA plate.

### (1) Anti-E2 rabbit monoclonal antibody

The anti-E2 rabbit monoclonal antibody used was the same antibody as in Example 1.

### (2) Anti-immunocomplex mouse monoclonal antibody

The anti-immunocomplex mouse monoclonal antibody used was the same antibody as in Example 1.

### (3) Measurement

A plate reader (TECAN, infinite F500) was used for measurement of E2 by the following method.

Anti-immunocomplex mouse monoclonal antibody was prepared as solid phase on a 96-well microplate (black, Greiner) in an amount of 0.25 µg/mL (carbonate buffer (0.05 M, pH 9.6)). Blocking treatment was then carried out with 1% skim milk/PBS. Using a separate plate, an E2 dilution series (2x dilution from 5,000 pg/mL final concentration) was prepared, and alkaline phosphatase-labeled anti-E2 rabbit monoclonal antibody was added to a final concentration of 1 mA (absorbance at 280 nm), before immediate dispensing into an anti-immunocomplex mouse monoclonal antibody-immobilized 96-well microplate at 100 µL/well. After incubation for 10 minutes, it was washed using a plate washer, 4-MUP (4-methylumbelliferyl phosphate, Merck) was added as enzyme substrate and the fluorescence intensity was measured after 30 minutes.

Table 3 below shows the measurement results for fluorescence intensity in Example 3 and Comparative Example 1. For easier comparison, Table 4 and Fig. 4 show the S/N (signal/noise) ratio calculation results. For all E2 concentrations, S/N was larger in Example 3 (anti-E2 rabbit monoclonal antibody as solid phase antibody), confirming that measurement sensitivity was higher when using anti-E2 rabbit monoclonal antibody as the solid phase antibody, even with the same antibody combination.

**[Table 3]**

| | | | | | | | | | | | |
|---|---|---|---|---|---|---|---|---|---|---|---|
| E2 concentration (pg/mL) | 0 | 10 | 20 | 39 | 78 | 156 | 313 | 625 | 1,250 | 2,500 | 5,000 |
| Example 3 (anti-E2 antibody solid phase) | 2,026 | 4,461 | 6,419 | 9,493 | 14,019 | 19,296 | 23,181 | 23,613 | 24,534 | 24,459 | 25,828 |
| Comp. Ex. 1 (anti-immunocomplex antibody solid phase) | 2,044 | 2,577 | 3,250 | 3,766 | 9,560 | 12,350 | 15,434 | 14,531 | 13,968 | 14,008 | 13,419 |

**[Table 4]**

| | | | | | | | | | | |
|---|---|---|---|---|---|---|---|---|---|---|
| E2 concentration (pg/mL) | 10 | 20 | 39 | 78 | 156 | 313 | 625 | 1,250 | 2,500 | 5,000 |
| Example 3 (anti-E2 antibody solid phase) | 2.2 | 3.2 | 4.7 | 6.9 | 9.5 | 11.4 | 11.7 | 12.1 | 12.1 | 12.7 |
| Comp. Ex. 1 (anti-immunocomplex antibody solid phase) | 1.3 | 1.6 | 1.8 | 4.7 | 6.0 | 7.6 | 7.1 | 6.8 | 6.9 | 6.6 |

### [Example 4]

An Example of the present invention will now be described in detail, for a method using rabbit monoclonal antibody for digoxin (Dig) and anti-immunocomplex mouse monoclonal antibody for the immune complex, with the understanding that the Example is not intended to be restrictive on the invention.

### (1) Anti-Dig rabbit monoclonal antibody

The anti-Dig rabbit monoclonal antibody was obtained by the method described in Japanese Unexamined Patent Publication No. 2009-240300.

### (2) Anti-immunocomplex mouse monoclonal antibody

The antibody for Dig/anti-Dig rabbit monoclonal antibody immune complex (anti-immunocomplex mouse monoclonal antibody) was obtained by the method described in Japanese Patent Publication No. 6031944.

### (3) Measurement

Automatic analysis was carried out by the following method using a fully automatic chemiluminescent enzyme immunoassay device (AIA-CL2400 by Tosoh Corp.).

### (3-1) Preparation of reagent cup

A cup having two cells as shown in Fig. 1 was used to prepare a reagent cup for use in automatic analysis. A solution containing microparticles immobilizing anti-Dig rabbit monoclonal antibody was dispensed in an amount of 50 µL into the microparticle cell. A solution containing alkaline phosphatase-labeled anti-immunocomplex mouse monoclonal antibody was dispensed in an amount of 75 µL into the conjugate cell. This was sealed with aluminum to obtain a reagent cup for Dig assay.

### (3-2) Measurement

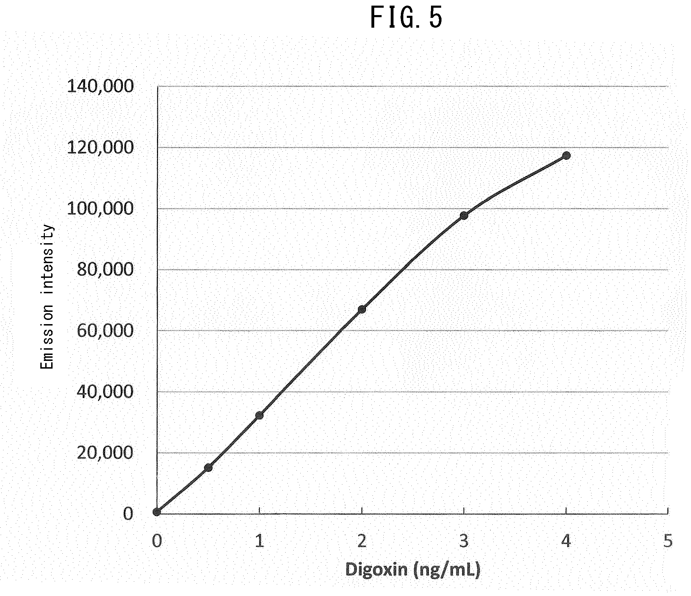

The Dig-assay reagent cup prepared in (3-1) was set in the fully automatic chemiluminescent enzyme immunoassay device (AIA-CL2400 by Tosoh Corp.) and measurement was conducted for 6 different samples (call to cal6) of known Dig concentration to construct a calibration curve. For the measurement, 10 µL of a sample of known concentration and 40 µL of a diluent were dispensed into the microparticle cell of the Dig-assay reagent cup and reacted for 5 minutes (first-order reaction), after which washing was performed with a cleaning fluid (B/F separation) and the reagent in the conjugate cell (50 µL) was transferred to the microparticle cell and reacted (secondary reaction, 3 minutes). After completion of the secondary reaction, a second washing was performed with cleaning fluid. Enzyme substrate was then added and the emission intensity was measured. The measurement results are shown in Table 5 and Fig. 5.

The emission intensity was confirmed to increase in proportion to Dig concentration, making it possible to construct a Dig assay system using anti-immunocomplex antibody.

**[Table 5]**

| | | | | | | |
|---|---|---|---|---|---|---|
| Dig concentration (ng/mL) | 0.0 | 0.5 | 1.0 | 2.0 | 3.0 | 4.0 |
| Emission intensity (cps) | 657 | 15,225 | 32,354 | 66,991 | 97,732 | 117,363 |

These results confirmed that Dig can be detected in an assay system using anti-hapten rabbit monoclonal antibody as a solid phase antibody and anti-immunocomplex antibody as a labeled antibody.

### [Example 5]

An Example of the invention will now be described in detail, for rabbit monoclonal antibody for estradiol (E2) and anti-immunocomplex mouse monoclonal antibody for the immune complex, and an absorption antibody (EE2B-18) added to reduce cross-reactivity, with the understanding that this Example is not restrictive on the invention.

### (1) Anti-E2 rabbit monoclonal antibody

The anti-E2 rabbit monoclonal antibody used was the same antibody as in Example 1.

### (2) Anti-immunocomplex mouse monoclonal antibody

The anti-immunocomplex mouse monoclonal antibody used was the same antibody as in Example 1.

### (3) Absorption antibody (antibody for ethynylestradiol (EE2))

The absorption antibody, i.e. antibody for the E2 analog EE2 (EE2B-18) was obtained by the following method.

### (3-1) Immunization of animals

The immunized animals used were six 5-week-old female mice. The antigen used was 1,3,5(10)-estratrien-17-α-ethynyl-3,17-beta-diol-6-one-6-carboxymethyloxime:BSA (Steraloids Co.), and an emulsion comprising the antigen solution and an adjuvant in equal amounts was prepared and used to immunize the mice 4 times at 1 week intervals. The immunization amount was 50 µg of antigen per mouse. The adjuvant used was Freund's complete adjuvant for the first inoculation, and Freund's incomplete adjuvant for the second and subsequent inoculations.

### (3-2) Confirmation of antibody titer

The increase in antibody titer was confirmed by ELISA as described below.

### (3-2-1)

EE2 chemically bound to KLH (KLH-EE2) was immobilized on an ELISA plate at 1 µg/mL and blocked with 1% skim milk solution.

### (3-2-2)

The obtained mouse antiserum was used to prepare a dilution series (2x dilution) from 1000x dilution and reacted with the KLH-EE2 in solid-phase on the ELISA plate.

### (3-2-3)

After B/F (Bound/Free) separation, α-Mouse IgG-ALP (Merck) was added to the plate as an alkaline phosphatase (ALP)-labeled antibody, and reacted with the mouse antibody on the plate.

### (3-2-4)

After B/F separation of the unreacted ALP-labeled antibody, the ALP substrate 4-methylumbelliferylphosphoric acid (4-MUP) was dispensed into the plate, the fluorescence intensity was measured to detect it, and the mice with increased antibody titer were selected.

### (3-3) Creation of absorption antibody-producing hybridomas

Antibody producing cells were prepared from the mice selected in (3-2) by the following method.
(3-3-1) Spleens were extracted from mice with increased antibody titer, and spleen cells were prepared by an established method. The prepared spleen cells were fused with mouse myeloma cells by electrical fusion to create hybridomas.
(3-3-2) The fused hybridoma suspensions were suspended in E-RDF medium (Kyokuto Pharmaceutical Industrial Co., Ltd.) containing 10% FCS (Fetal calf serum) and 1 × HAT (Sigma), and then spread on a microtiter plate and cultured for 8 days, after which the culture supernatants were obtained.

### (3-4) Screening of absorption antibody-producing hybridomas

The absorption antibody preferably has high reactivity with EE2 and low reactivity with E2. For screening of the absorption antibody of the invention, therefore, it is sufficient to select antibodies that selectively react with EE2 even in the presence of E2. Specifically, the screening was carried out by ELISA in the following manner.

### (3-4-1)

KLH-EE2 was immobilized on an ELISA plate at 1 µg/mL and blocked with 1% skim milk solution.

### (3-4-2)

The culture supernatant was reacted with KLH-EE2 in solid phase on ELISA plates in the presence or in the absence of E2.

### (3-4-3)

After B/F separation, α-Mouse IgG-ALP (Merck) was added to the plate as ALP-labeled antibody, and reacted with the mouse antibody on the plate.

### (3-4-4)

The unreacted ALP-labeled antibody was removed by B/F separation, after which the ALP substrate 4-MUP was dispensed into the plate and detected by fluorescence intensity measurement, and hybridomas producing antibodies that reacted with EE2 in the presence of E2 were selected out.

### (3-5) Production and purification of absorption antibodies

The absorption antibody-producing hybridomas selected in (3-4) were cultured in E-RDF medium (Kyokuto Pharmaceutical Industrial Co., Ltd.) containing 10% FCS, and the culture supernatants were obtained. The antibodies in the culture supernatants were concentrated by ammonium sulfate precipitation and a TSKgel Ether-5pw column was used for purification to obtain an absorption antibody (EE2B-18).

### (4) Automatic analysis

Automatic analysis was carried out by the following method using a fully automatic chemiluminescent enzyme immunoassay device (AIA-CL2400 by Tosoh Corp.).

### (4-1) Preparation of reagent cup

A cup having two cells was used to prepare a reagent cup for use in automatic analysis. Absorption antibody (EE2B-18) was added at 10 µg/mL to a solution containing microparticles immobilizing anti-E2 rabbit monoclonal antibody, and the mixture was dispensed into the microparticle cell. A solution containing β-estradiol-6-one-6-(O-carboxymethyloxime) was dispensed into the conjugate cell together with alkaline phosphatase-labeled anti-immunocomplex antibody, with reference to PTL 1. The solution was lyophilized and sealed with aluminum to prepare an E2-assay reagent cup.

### (4-2) Measurement

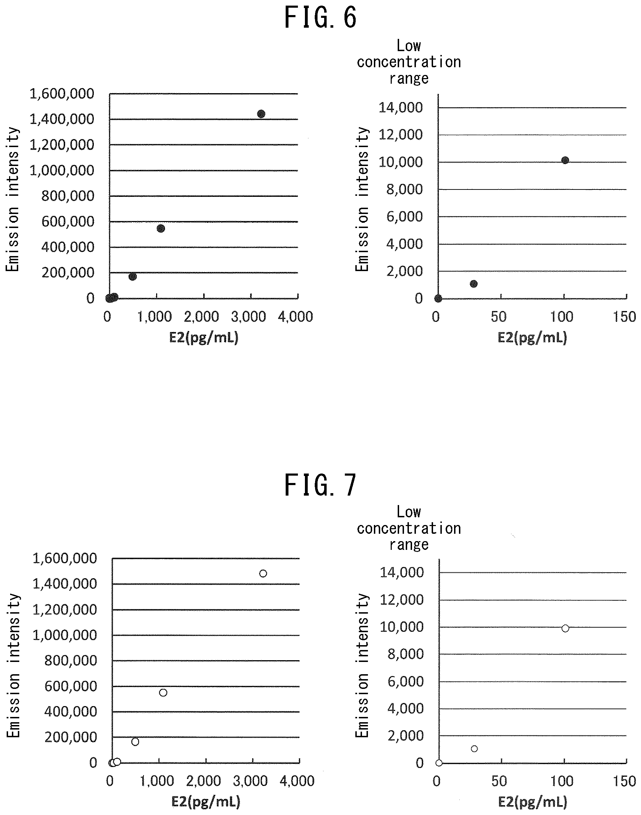

The E2-assay reagent cup prepared in (4-1) was set in the fully automatic chemiluminescent enzyme immunoassay device (AIA-CL2400 by Tosoh Corp.) and measurement was conducted for 6 different samples (call to cal6) of known E2 concentration to construct a calibration curve. The measurement was carried out in the same manner as (3-2) of Example 1. The measurement results are shown in Fig. 6. The emission intensity was confirmed to increase in proportion to E2 concentration, making it possible to construct an automatic analysis system for E2 using anti-immunocomplex antibody when in the presence of absorption antibody.

### (4-3) Evaluation of cross-reactivity

A solution prepared to 10 ng/mL EE2 was used for measurement of the emission intensity in the same manner as (4-2), using the E2-assay reagent cup prepared in (4-1) and AIA-CL2400. The cross-reaction rate was calculated from the calibration curve prepared in (4-2). The results are shown in Table 6.

### [Example 6]

An example containing no absorption antibody (EE2B-18) in the E2-assay reagent cup prepared in Example 5 will now be described as Example 6.

### (1) E2-assay reagent cup containing no absorption antibody

An E2-assay reagent cup was produced in the same manner as Example 5(4-1), but without addition of absorption antibody.

### (2) Measurement

The E2-assay reagent cup prepared in (1) was set in an AIA-CL2400 and measurement was conducted for 6 different samples (call to cal6) of known E2 concentration to construct a calibration curve. The measurement was carried out in the same manner as (3-2) of Example 1. The measurement results are shown in Fig. 7. It was confirmed that with or without absorption antibody, there was no significant change in the calibration curve with respect to Fig. 6 (Example 5).

### (3) Evaluation of cross-reactivity

Measurement was carried out in the same manner as Example 5(4-2) with an AIA-CL2400, using the E2-assay reagent cup prepared in (1) and a solution prepared to 10 ng/mL EE2. The cross-reaction rate was calculated from the calibration curve prepared in (2).

The cross-reaction rates measured in Example 5 and Example 6 are shown in Table 6.

**[Table 6]**

| | Example 5 | Example 6 |
|---|---|---|
| Cross-reaction rate (%) | 9.7 | 15.4 |

The results for the cross-reaction rates in Table 6 indicate that the presence of absorption antibody (EE2B-18) can drastically improve cross-reaction. This confirmed that the presence of absorption antibody can inhibit cross-reaction in an assay system using anti-immunocomplex antibody.

### [Example 7]

An Example of the invention will now be described in detail, for rabbit monoclonal antibody for estradiol (E2) and anti-immunocomplex mouse monoclonal antibody for the immune complex, and an absorption antibody added to reduce cross-reactivity, with the understanding that this Example is not restrictive on the invention.

### (1) Anti-E2 rabbit monoclonal antibody

The anti-E2 rabbit monoclonal antibody was obtained by the method described in Japanese Unexamined Patent Publication No. 2009-240300.

### (2) Anti-immunocomplex mouse monoclonal antibody

The antibody for E2/anti-E2 rabbit monoclonal antibody immune complex (anti-immunocomplex mouse monoclonal antibody) was obtained by the method described in Japanese Patent Publication No. 6031944.

### (3) Absorption antibody (antibody for ethynylestradiol (EE2))

The antibody for the E2 analog EE2 was prepared by the same method as Example 5 as a different antibody from EE2B-18.

### (4) Automatic analysis

Automatic analysis was carried out by the following method using a fully automatic chemiluminescent enzyme immunoassay device (AIA-CL2400 by Tosoh Corp.).

### (4-1) Preparation of reagent cup

A cup having two cells was used to prepare a reagent cup for use in automatic analysis.

Absorption antibody was added at 10 µg/mL to a solution containing microparticles immobilizing anti-E2 rabbit monoclonal antibody, and the mixture was dispensed into the microparticle cell. A solution containing β-estradiol-6-one-6-(O-carboxymethyloxime) was dispensed into the conjugate cell together with alkaline phosphatase-labeled anti-immunocomplex antibody, with reference to PTL 1. The solution was lyophilized and sealed with aluminum to prepare an E2-assay reagent cup.

### (4-2) Measurement

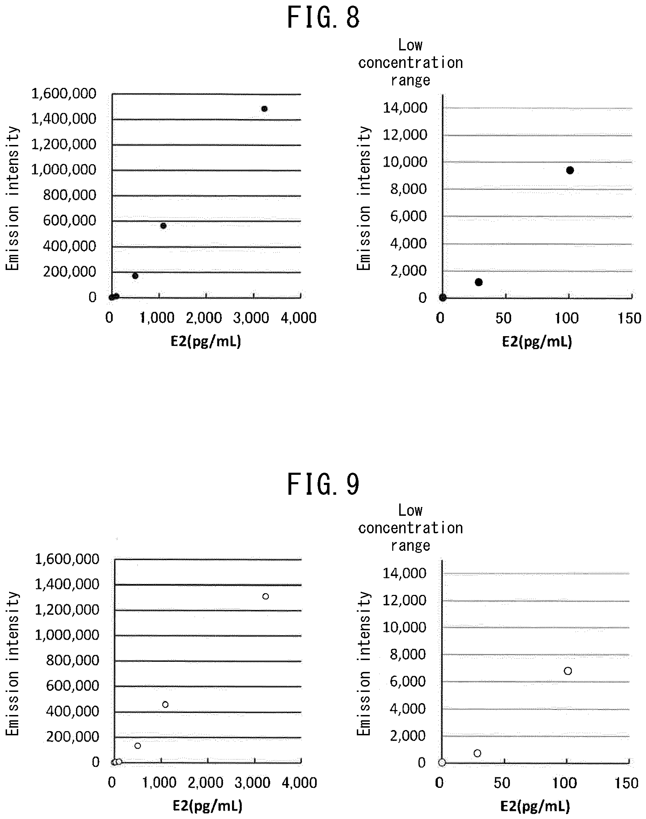

The E2-assay reagent cup prepared in (4-1) was set in the fully automatic chemiluminescent enzyme immunoassay device (AIA-CL2400 by Tosoh Corp.) and measurement was conducted for 6 different samples (call to cal6) of known E2 concentration to construct a calibration curve. The measurement results are shown in Fig. 8. The emission intensity was confirmed to increase in proportion to E2 concentration, making it possible to construct an automatic analysis system for E2 using anti-immunocomplex antibody when in the presence of absorption antibody.

### (4-3) Evaluation of cross-reactivity

A solution prepared to 10 ng/mL EE2 was used for measurement of the emission intensity using the E2-assay reagent cup prepared in (4-1) and AIA-CL2400. The cross-reaction rate was calculated from the calibration curve prepared in (4-2). The results are shown in Table 7.

### [Example 8]

An example containing no absorption antibody in the E2-assay reagent cup prepared in Example 7 will now be described as Example 8.

### (1) E2-assay reagent cup containing no absorption antibody

An E2-assay reagent cup was produced in the same manner as Example 7(4-1), but without addition of absorption antibody.

### (2) Measurement

The E2-assay reagent cup prepared in (1) was set in an AIA-CL2400 and measurement was conducted for 6 different samples (call to cal6) of known E2 concentration to construct a calibration curve. The measurement results are shown in Fig. 9. It was confirmed that with or without absorption antibody, there was no significant change in the calibration curve with respect to Fig. 8 (Example 7).

### (3) Evaluation of cross-reactivity

Measurement was carried out with an AIA-CL2400, using the E2-assay reagent cup prepared in (1) and a solution prepared to 10 ng/mL EE2. The cross-reaction rate was calculated from the calibration curve prepared in (2).

The cross-reaction rates measured in Example 7 and Example 8 are shown in Table 7.

**[Table 7]**

| | Example 7 | Example 8 |
|---|---|---|
| Cross-reaction rate (%) | 6.0 | 14.9 |

The results for the cross-reaction rates indicate that the presence of absorption antibody can drastically improve cross-reaction. This confirmed that the method of the invention can inhibit cross-reaction in an assay system using anti-immunocomplex antibody.

The present invention has been described in detail using specific embodiments, but it will be apparent to a person skilled in the art that various modifications and alterations may be employed such as are within the spirit and scope of the invention.

The entire contents of the specifications, claims, drawings and abstracts of Japanese Patent Application No. 2019-209407 filed on November 20, 2019, Japanese Patent Application No. 2019-216521 filed on November 29, 2019, Japanese Patent Application No. 2020-042915 filed on March 12, 2020, Japanese Patent Application No. 2020-100842 filed on June 10, 2020 and Japanese Patent Application No. 2020-138101 filed on August 18, 2020, are incorporated by reference in the present disclosure.

## Claims

1. An immunoassay method using:
• an anti-hapten rabbit monoclonal antibody immobilized on a water-insoluble carrier, and
• a labeled anti-immunocomplex antibody,
wherein the method comprises the following steps (i) to (iii):
(i) a step of reacting the anti-hapten rabbit monoclonal antibody immobilized on the water-insoluble carrier with a hapten in a solution to be measured,
(ii) a step of reacting the labeled anti-immunocomplex antibody with the hapten-anti-hapten rabbit monoclonal antibody immune complex, and
(iii) a step of detecting the signal from the label.

2. The method according to claim 1, wherein steps (i), (ii) and (iii) are carried out in that order, and the method has washing steps between steps (i) and (ii) and between steps (ii) and (iii).

3. The method according to claim 1 or 2, wherein the anti-immunocomplex antibody is a mouse monoclonal antibody.

4. The method according to any one of claims 1 to 3, wherein the label is alkaline phosphatase.

5. The method according to any one of claims 1 to 4, wherein the hapten is estradiol, thyroxine or digoxin.

6. The method according to any one of claims 1 to 5, wherein step (i) is carried out in the presence of an absorption antibody.
